# EUROPEAN PATENT APPLICATION

(11) **EP 3 875 096 A1**
(43) Date of publication of application: **08.09.2021**
(21) Application number: 19880997.2
(22) Date of filing: 05.11.2019
(51) Int. Cl.: A61K 35/19, A61K 9/00, A61K 35/18, A61K 47/22, A61K 47/26, A61P 7/08

(54) **METHOD FOR PRODUCING BLOOD PREPARATION AND BLOOD PREPARATION**

(30) Priority: 07.11.2018 JP 2018209374
(71) Applicant: TERUMO Kabushiki Kaisha, Tokyo 151-0072 (JP)
(72) Inventor: TAKEDA, Norihiko, Fujinomiya-shi, Shizuoka 418-0004 (JP)
(74) Representative: TBK
(86) International application number: PCT/JP2019/043303
(87) International publication number: WO 2020/095901

(57) **Abstract**

An object of the invention is to provide a means for accomplishing inactivation of a pathogen in a blood sample containing red blood cells and platelets together, as well as sufficiently reducing the hemolysis rate of the red blood cell product finally obtained.

The means is to irradiate ultraviolet ray to a blood sample containing red blood cells and platelets in the presence of a hemolysis inhibitor and a surfactant having an HLB value of 13 or greater and having the number of oxyethylene groups of a hydrophilic portion in the molecular structure of 20 or greater when producing a blood product containing red blood cells and platelets.

## Description

### TECHNICAL FIELD

The present invention relates to a method for producing a blood product and a blood product.

### BACKGROUND ART

In recent years, blood component transfusion in which components of blood (whole blood) obtained by blood donation or the like are separated to provide only components required by a patient has been performed in blood transfusion. According to the blood component transfusion, it is possible to alleviate a burden on the patient's circulatory system and other side effects, and effective utilization of the donated blood is achieved.

In general, blood (whole blood) collected in a blood bag from a donor by blood donation is centrifuged into necessary blood products (a red blood cell product, a platelet product, and a blood plasma product). Each of the blood products is transferred to each of a plurality of bags, and a pathogen such as a virus is inactivated in each blood product. For example, JP 2004-081692 A discloses a technique in which a photoactive agent (pathogen inactivating agent) is added to a bag housing a blood product, and light having a predetermined wavelength is irradiated to the pathogen inactivating agent to activate the pathogen inactivating agent, thereby inactivating a pathogen. However, in the technique described in JP 2004-081692 A, blood collected in a blood bag is separated into a plurality of blood products, and the pathogen in each of the blood products is then subjected to an inactivating treatment. This makes it necessary to perform the same number of inactivating treatments as that of the blood products, which disadvantageously takes a lot of time and cost.

A technique of irradiating ultraviolet ray to a blood sample containing red blood cells and platelets together in the presence of a photosensitizer to inactivate a pathogen has been proposed. However, in this case, disadvantageously, most of light necessary for activating the photosensitizer is absorbed by red blood cell components in the blood sample, so that it may not be effectively utilized for inactivating the pathogen. On the other hand, if a sufficient amount of light for inactivating a pathogen in red blood cells is irradiated to the blood sample, red blood cell components and components other than the red blood cell components are damaged, so that the quality of a blood product obtained is also disadvantageously deteriorated.

In order to solve these problems, WO 2008/71541 (JP 2010-535235 A) discloses a technique of irradiating ultraviolet ray to a sample containing red blood cells and platelets together in the presence of an alloxazine photosensitizer such as riboflavin before being separated into blood products to inactivate a pathogen in the blood sample.

Incidentally, for example, the product standard of red blood cell products in Europe is set to satisfy the condition of "the hemolysis rate is 0.8% or less" over a storage period of 6 weeks or greater. At present, the red blood cell product supplied to the market expires the expiration date of 6 weeks, and is discarded. In Europe alone, there is a growing consciousness of "the fact that a significant amount of valuable blood resources collected by blood donation has been discarded must be remedied" (see Ayyalil et al., Transfusion 2017; 57(12): 2870-7). Therefore, if the period during which the red blood cell product satisfies the above condition can be extended, such a loss of valuable value can also be greatly reduced, which is very preferable not only from an economic aspect but also from an ethical viewpoint.

### SUMMARY OF INVENTION

### Technical Problem

According to the studies of the present inventors, it has been found that even by the technique described in WO 2008/71541 (JP 2010-535235 A), inactivation of a pathogen in a blood sample containing red blood cells and platelets together cannot be accomplished, as well as sufficiently reducing the hemolysis rate of the red blood cell product finally obtained.

Therefore, an object of the present invention is to provide a means for accomplishing inactivation of a pathogen in a blood sample containing red blood cells and platelets together, as well as sufficiently reducing the hemolysis rate of the red blood cell product finally obtained.

### Solution to Problem

The present inventors have conducted intensive studies in order to solve the above problems. As a result, the present inventors surprisingly found that the above problems can be solved by irradiating ultraviolet ray to a blood sample containing red blood cells and platelets together in the presence of a hemolysis inhibitor and a predetermined surfactant. Based on this finding, the present inventors have completed the present invention.

That is, according to an aspect of the present invention, there is provided a method for producing a blood product, including: irradiating ultraviolet ray to a blood sample including red blood cells, platelets, a hemolysis inhibitor, and a surfactant having an HLB value of 13 or greater and having the number of oxyethylene groups of a hydrophilic portion in the molecular structure of 20 or greater.

According to another aspect of the present invention, there is provided a blood product including red blood cells, platelets, a hemolysis inhibitor and a surfactant having an HLB value of 13 or greater and having the number of oxyethylene groups of a hydrophilic portion in the molecular structure of 20 or greater, wherein a pathogen in the blood product is inactivated.

Furthermore, according to still another aspect of the present invention, there is provided a storage solution for a blood cell including a hemolysis inhibitor and a surfactant having an HLB value of 13 or greater and having the number of oxyethylene groups of a hydrophilic portion in the molecular structure of 20 or greater, wherein the storage solution is used by being mixed with the blood sample in the method for producing according to the aspect described above.

### BRIEF DESCRIPTION OF DRAWINGS

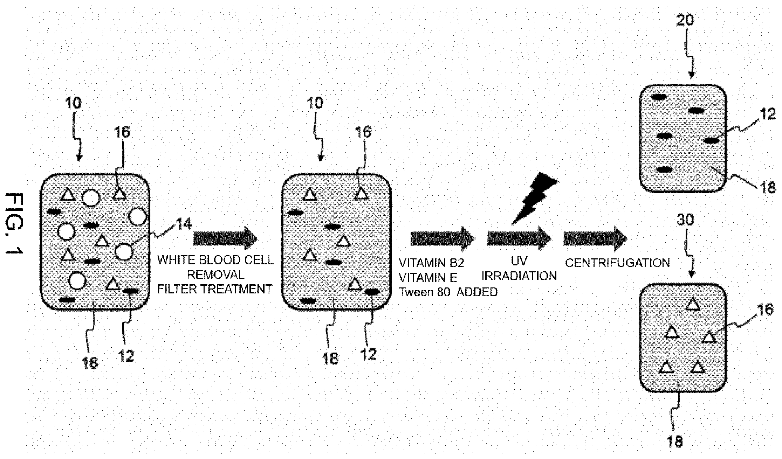

FIG. 1 is an explanatory view for explaining an embodiment of a method for producing a blood product according to the present invention.

### DESCRIPTION OF EMBODIMENTS

Hereinafter, embodiments of the present invention will be described with reference to the accompanying drawings.

### <<Method for Producing Blood Product>>

An aspect of the present invention relates to a method for producing a blood product, including: irradiating ultraviolet ray to a blood sample including red blood cells, platelets, a hemolysis inhibitor, and a surfactant having an HLB value of 13 or greater and having the number of oxyethylene groups of a hydrophilic portion in the molecular structure of 20 or greater. According to the method for producing a blood product according to the present invention, it is possible to accomplish inactivation of a pathogen in a blood sample containing red blood cells and platelets together, as well as sufficiently reducing the hemolysis rate of the red blood cell product finally obtained.

FIG. 1 is an explanatory view for explaining an embodiment of a method for producing a blood product according to the present invention. In the method for producing a blood product according to the embodiment shown in FIG. 1, first, a whole blood sample 10 is prepared, which contains a blood plasma 18 containing red blood cells 12, white blood cells 14, and platelets 16. Next, the whole blood sample 10 is treated using a white blood cell removal filter to remove the white blood cells 14. Vitamin B₂ (riboflavin), vitamin E (tocopherol acetate) as a hemolysis inhibitor, and Tween (registered trademark, the same applies hereinafter) 80 (polyoxyethylene sorbitan monooleate) as a surfactant are then added to the sample. After these are added, ultraviolet ray is irradiated to the sample to inactivate a pathogen in the sample. A red blood cell product 20 containing the blood plasma 18 containing the red blood cells 12 and a platelet product 30 containing the blood plasma 18 containing the platelets 16 are obtained by a centrifugation treatment.

Hereinafter, the present invention will be described in more detail.

### [Blood Sample]

In the producing method according to the present aspect, first, a blood sample is prepared. The blood sample contains red blood cells and platelets, and also contains a hemolysis inhibitor and a predetermined surfactant which will be described later. The blood sample may be a whole blood sample, or may be derived from the whole blood sample. However, from the viewpoint of reducing the occurrence of side effects during blood transfusion, the blood sample is preferably derived from one from which white blood cells were removed such that the number thereof was reduced to about 1×10⁶ or less per bag by subjecting a whole blood sample to a treatment for removing the white blood cells. As a method for removing the white blood cells from the whole blood sample, a conventionally known method can be used, and examples thereof include a method using a white blood cell removal filter when collecting the whole blood sample at blood donation and the like.

### [Hemolysis Inhibitor]

The hemolysis inhibitor is for preventing impairment of red blood cell membranes via an antioxidation effect, affinity for the lipid of the red blood cell membranes, and the like. Specifically, vitamin E or an unsaturated linear hydrocarbon selected from the group consisting of 7-tetradecene, 8-octadecene, 9-eicosene, and squalene, is preferable. Examples of the vitamin E include tocopherols such as α-tocopherol, β-tocopherol, γ-tocopherol, and δ-tocopherol, and tocotrienols such as α-tocotrienol, β-tocotrienol, γ-tocotrienol, and δ-tocotrienol, and the like, among which α-tocopherol is preferable. The vitamin E may be ester compounds of these tocopherols or tocotrienols, and is preferably compounds of ester acetate, specifically a tocopherol acetate.

Since the concentration of the hemolysis inhibitor in the red blood cell product is preferably 25 to 100 ppm, the concentration of the hemolysis inhibitor in the blood sample is preferably 75 to 300 ppm when added together with a surfactant. If the concentration is 75 ppm or greater, the impairment of the red blood cell membranes can be sufficiently prevented. Specifically, the hemolysis preventive rate is likely to decrease. In other words, the amount of Hb in the blood plasma is likely to increase. If the concentration is 300 ppm or less, an increase in cost due to saturation of an effect of preventing the impairment of the red blood cell membranes can be prevented.

### [Surfactant]

The surfactant has an effect for promoting uniform dispersion of a hemolysis inhibitor in a blood sample and coating of red blood cell membranes with the hemolysis inhibitor, and itself can prevent impairment of the red blood cell membranes. Therefore, the surfactant essentially has an HLB value of 13 or greater. The HLB value of the surfactant is preferably 13 to 20, and the number of oxyethylene groups (EO counts) of a hydrophilic portion in the molecular structure is 20 or greater, and preferably 20 to 40. Preferable examples of the molecular structure of the surfactant include a molecular structure having a hydrophilic portion composed of polyoxyethylene (PEO), and a molecular structure having a hydrophilic portion composed of polyoxyethylene sorbitan (PEO sorbitan).

Here, if the HLB value of the surfactant is less than 13, the surfactant may not be sufficiently dispersed in the blood sample, so that the impairment of the red blood cell membranes may not be able to be prevented. If the EO counts are less than 20, the molecular weight of the hydrophilic portion of the surfactant is low, so that the impairment of the red blood cell membranes may not be able to be prevented. If the HLB value exceeds 20 or the EO counts exceed 40, the prevention of the impairment of the red blood cell membranes is not remarkably improved, and cost is apt to increase. The HLB value is obtained by measuring via Griffin's method and calculating via the formula: HLB value = (20 × sum of formula weight of hydrophilic portion)/(molecular weight of surfactant).

Examples of the surfactant having a hydrophilic portion composed of polyoxyethylene (PEO) include, for example, polyoxyethylene oleyl ether (EMULGEN (registered trademark) 430), and polyoxyethylene lauryl ether (EMULGEN 130K) and the like.

Examples of the surfactant having a hydrophilic portion composed of polyoxyethylene sorbitan (PEO sorbitan) include, for example, polyoxyethylene sorbitan monooleate (Tween (registered trademark) 80), polyoxyethylene sorbitan monostearate (Tween 60), and polyoxyethylene sorbitan monolaurate (Tween 20) and the like. Among them, a surfactant having a hydrophilic portion composed of polyoxyethylene sorbitan (PEO sorbitan) is preferable, and polyoxyethylene sorbitan monooleate (Tween (registered trademark) 80) is particularly preferable.

Since the concentration of the surfactant in the red blood cell product is preferably 100 to 300 ppm, the concentration of the surfactant in the blood sample when added together with the hemolysis inhibitor is preferably 300 to 900 ppm. If the concentration is 300 ppm or greater, the impairment of the red blood cell membranes can be sufficiently prevented. Specifically, a decrease in the hemolysis preventive rate can be prevented. In other words, the amount of Hb in the blood plasma is less likely to increase. If the concentration is 900 ppm or less, the impairment of the red blood cell membranes can be sufficiently prevented, and an increase in cost can also be prevented.

The blood sample may further contain additives other than those described above. For example, the blood sample may further contain an alloxazine photosensitizer such as riboflavin as disclosed in WO 2008/71541 (JP 2010-535235 A).

### [Step for Irradiating Ultraviolet Ray]

The method for producing a blood product according to the present aspect includes a step for irradiating ultraviolet ray to the blood sample prepared above (containing red blood cells, platelets, a hemolysis inhibitor, and a surfactant having an HLB value of 13 or greater and having the number of oxyethylene groups of a hydrophilic portion in the molecular structure of 20 or greater) .

A specific embodiment of the ultraviolet ray used in the present step is not particularly limited. As an example, the peak wavelength of the ultraviolet ray is preferably within a range of 200 nm or greater to 400 nm or less, more preferably within a range of 290 nm or greater to 330 nm or less, or within a range of 360 nm or greater to 380 nm or less, and particularly preferably within a range of 300 nm or greater to 315 nm or less. Here, if the peak wavelength is within a range of 300 nm or greater to 315 nm or less, the red blood cells can be suitably transmitted.

A means for irradiating the ultraviolet ray is also not particularly limited, and a conventionally known technique can be appropriately adopted. Examples thereof include a low-pressure mercury lamp, a medium-pressure mercury lamp, a high-pressure mercury lamp, an ultra-high pressure mercury lamp, a chemical lamp, a black light lamp, a microwave-excited mercury lamp, and a metal halide lamp and the like. In addition, Mirasol (registered trademark) system available from Terumo BCT, Inc., Lakewood (Colorado) may be used. An ultraviolet ray irradiation amount (cumulative light amount) is not particularly limited, but it is preferably 40 J/mL RBC or greater to 120 J/mL RBC or less, and more preferably 60 J/mL RBC or greater to 90 J/mL RBC or less from the viewpoint of accomplishing both sufficient inactivation of the pathogen and particularly minimization of damage to red blood cell components.

### [Other Steps]

In the method for producing a blood product according to the present invention, other steps may be carried out after the step for irradiating ultraviolet ray to the blood sample as described above is carried out. For example, the method may further include separating the blood sample irradiated with the ultraviolet ray into a red blood cell product and a platelet product after the irradiation of the ultraviolet ray. A specific technique for separating the blood sample irradiated with the ultraviolet ray into the red blood cell product and the platelet product is not particularly limited, and a generally used centrifugation treatment can be adopted as a preferable technique. A person skilled in the art can appropriately determine specific devices and conditions for the centrifugation treatment in consideration of the physical properties of each of the products, and the like.

### <<Blood Product>>

According to the method for producing a blood product according to the above-described aspect, a blood product is obtained. Thus, another aspect of the present invention also provides a blood product. The blood product provided in this manner contains red blood cells, platelets, a hemolysis inhibitor, and a surfactant having an HLB value of 13 or greater and having the number of oxyethylene groups of a hydrophilic portion in the molecular structure of 20 or greater. The blood product is also characterized in that a pathogen is inactivated. In the blood product according to the present invention, "the pathogen is inactivated" means that the inactivation effect of the pathogen is not significantly low as compared with a blood product obtained by performing an inactivating treatment without using the hemolysis inhibitor and the surfactant according to the present invention. Here, whether or not the pathogen in the blood product is inactivated can be confirmed by, for example, a plaque assay.

### <<Storage Solution for Blood Cell>>

As described above, for the production of the blood product according to the present invention, the case where the hemolysis inhibitor and the predetermined surfactant are directly added into the blood sample has been described as an example, but the present invention is not limited to such an embodiment. For example, the hemolysis inhibitor and the predetermined surfactant may be dissolved in a suitable solvent in advance of the preparation of a storage solution for a blood cell. By adding the storage solution for a blood cell into the blood sample containing red blood cells and platelets, the red blood cells and the platelets may be mixed with the hemolysis inhibitor and the predetermined surfactant. As the solvent for preparing the storage solution for a blood cell, a conventional known storage solution used for the long-term storage of the blood product is used. Examples of the storage solution include ACD solution, CPD solution, MAP solution, SAGM solution, OPTISOL (registered trademark) (AS-5), ADSOL (AS-1), Nutricel (AS-3), PAGG-S, and SAGP-maltose and the like. Among them, the MAP solution, the SAGM solution, or the PAGG-S is preferable. Here, the MAP solution is a mixed solution containing mannitol, glucose, adenine, a phosphate, a citrate, and sodium chloride. The SAGM solution is a mixed solution containing mannitol, glucose, adenine, and sodium chloride. Furthermore, the PAGG-S is a mixed solution containing sorbitol, glucose, adenine, guanosine, a phosphate, and sodium chloride.

The present invention can accomplish the inactivation of the pathogen in the blood sample containing the red blood cells and the platelets together, as well as sufficiently reducing the hemolysis rate of the red blood cell product finally obtained, as clearly demonstrated in Example to be described later. Such an effect is exhibited, whereby the expiration date of the red blood cell product can be particularly extended. As a result, a large loss of value caused by the disposal of the blood product due to expiration may be saved. The present invention can easily inactivate the pathogen in the red blood cell product, whereby a safe blood product can be provided to all patients requiring blood transfusion. In particular, in the Middle East and emerging countries, a ratio of blood contamination by pathogens such as hepatitis B virus (total treatment cost: about 800,000 yen/person) and HIV (annual treatment cost by anti-HIV drugs: about 3 million yen/person) is seriously 10 to 50%, but the advantage of allowing the supply of safe blood products to patients in these regions is immeasurable.

### Examples

The effects of the present invention will be described using the following Example and Comparative Example. However, the technical scope of the present invention is not limited to only the following Example.

### [Preparation of Blood Product]

### (Comparative Example)

First, a human-derived whole blood sample was prepared. White blood cell components were then removed from the whole blood sample using a white blood cell removal filter.

Then, the sample from which the white blood cell components were removed was infected with bacteriophage ϕX174 as a substitute microorganism in an amount of 5×10⁶ PFU. The infected sample was treated using Mirasol (registered trademark) system available from Terumo BCT, Inc., Lakewood (Colorado). Specifically, in accordance with the instructions attached to the system, vitamin B₂ (riboflavin) was added to the sample, and ultraviolet ray was then irradiated to the sample to inactivate a pathogen in the sample. The addition concentration of the vitamin B₂ (riboflavin) in this case was 140 ppm, and the irradiation condition of the ultraviolet ray was 80 J/mL RBC.

After the sample was treated by the Mirasol, the sample was subjected to a centrifugation treatment to be separated into a product containing a red blood cell component (red blood cell product) and a product containing platelets (platelet product). Thus, blood products (red blood cell product and platelet product) of the present Comparative Example were obtained.

### (Example)

Blood products (red blood cell product and platelet product) of the present Example were obtained in the same manner as in Comparative Example described above except that a hemolysis inhibitor (vitamin E; tocopherol acetate) and a surfactant (polyoxyethylene sorbitan monooleate); Tween (registered trademark) 80) were further added in conjunction with the addition of vitamin B₂ (riboflavin). In this case, the addition concentration of the hemolysis inhibitor was 50 ppm, and the addition concentration of the surfactant was 100 ppm.

### [Influence on Inactivating Effect of Pathogen]

For the red blood cell product obtained in each of Comparative Example and Example described above, the effect of reducing a pathogen (ϕX174) was compared with that before the inactivating treatment by the Mirasol system. Specifically, the amount of a pathogen (PFU/mL (plaque forming unit per sample unit volume)) before and after an inactivating treatment was calculated by a plaque assay, and an amount of reduction thereof was calculated as a log reduction value (log PFU/mL). For example, if the amount of the pathogen becomes 1/10, the log reduction value is "1", and if the amount of the pathogen becomes 1/100, the log reduction value is "2". These results are shown in Table 1 below. The results shown in Table 1 were obtained by performing the same test using seven samples in both Example and Comparative Example.

**[Table 1]**

| | Average value | Standard deviation |
|---|---|---|
| Example | 2.073 | 0.244 |
| Comparative Example | 2.070 | 0.070 |

The results shown in Table 1 confirmed that even when the present invention is applied (Example), the reduction effect of the pathogen by the irradiation of the ultraviolet ray is not adversely affected.

### [Influence on Hemolysis]

For the red blood cell product obtained in each of Comparative Example and Example described above, a change in a hemolysis rate was monitored over time. Specifically, for the red blood cell product obtained in each of Comparative Example and Example in addition to the sample before the inactivating treatment, the hemolysis rate was measured using an absorbance method on days 7, 14, 21, 28, 35, and 42 from day 0 immediately after the inactivating treatment. These results are shown in Table 2 below. The results shown in Table 2 are also obtained from the measurements on the same seven samples as those in Table 1 above in both Example and Comparative Example.

**[Table 2]**

| Comparative Example (N = 7) | | | | | | | | | Example (N = 7) | | | | | | |
|---|---|---|---|---|---|---|---|---|---|---|---|---|---|---|---|
| | Before treatment | Day 0 | Day 7 | Day 14 | Day 21 | Day 28 | Day 35 | Day 42 | Day 0 | Day 7 | Day 14 | Day 21 | Day 28 | Day 35 | Day 42 |
| Average value | 0.27 | 0.08 | 0.18 | 0.22 | 0.37 | 0.57 | 0.87 | 1.17 | 0.10 | 0.19 | 0.18 | 0.31 | 0.40 | 0.56 | 0.71 |
| Standard deviation | 0.21 | 0.04 | 0.12 | 0.13 | 0.21 | 0.30 | 0.48 | 0.61 | 0.07 | 0.12 | 0.09 | 0.14 | 0.20 | 0.32 | 0.39 |
| Conformity number of EU guideline | 7/7 | 7/7 | 7/7 | 7/7 | 7/7 | 6/7 | 3/7 | 2/7 | 7/7 | 7/7 | 7/7 | 7/7 | 7/7 | 6/7 | 5/7 |
| Conformity number of US guideline | 7/7 | 7/7 | 7/7 | 7/7 | 7/7 | 7/7 | 3/7 | 2/7 | 7/7 | 7/7 | 7/7 | 7/7 | 7/7 | 6/7 | 5/7 |

From the results shown in Table 2, in the red blood cell product of Comparative Example, up to day 21 after the inactivating treatment, all samples had a sufficiently low hemolysis rate for conforming to the European (EU) and US (US) guidelines. However, on day 28, one sample failed to satisfy the European guideline, and after day 35, a majority of samples failed to conform to the European and US guidelines.

In contrast, in the red blood cell product of Example, up to day 28 after the inactivating treatment, all samples had a sufficiently low hemolysis rate for conforming to the European and US guidelines. Also on days 35 and 42, most samples maintained a sufficiently low hemolysis rate for conforming to the European and US guidelines.

From the above, it is found that the inactivation of the pathogen in the blood sample containing red blood cells and platelets together can be accomplished as well as sufficiently reducing the hemolysis rate of the red blood cell product finally obtained according to the present invention. The mechanism by which such an effect is exhibited is presumed to be based on the fact that the hemolysis inhibitor and the surfactant according to the present invention exhibit an action of protecting the red blood cells in the inactivating treatment of the pathogen on the red blood cells (irradiation treatment with ultraviolet ray).

The present application is based on Japanese Patent Application No. 2018-209374 filed on November 7, 2018, and the disclosure content is incorporated by reference in its entirety.

### Reference Signs List

- 10: Whole blood sample
- 12: Red blood cells
- 14: White blood cells
- 16: Platelets
- 18: Blood plasma
- 20: Red blood cell product
- 30: Platelet product

## Claims

1. A method for producing a blood product, comprising:
irradiating ultraviolet ray to a blood sample comprising red blood cells, platelets, a hemolysis inhibitor, and a surfactant having an HLB value of 13 or greater and having the number of oxyethylene groups of a hydrophilic portion in the molecular structure of 20 or greater.

2. The method for producing according to claim 1, wherein the hemolysis inhibitor comprises Vitamin E.

3. The method for producing according to claim 1 or 2, wherein the surfactant comprises a polyoxyethylene sorbitan fatty acid ester.

4. The method for producing according to claim 3, wherein the surfactant comprises polyoxyethylene sorbitan monooleate (Tween (registered trademark) 80).

5. The method for producing according to any one of claims 1 to 4, wherein the blood sample is the one from which white blood cells were removed.

6. The method for producing according to any one of claims 1 to 5, further comprising:
separating the blood sample irradiated with the ultraviolet ray into a red blood cell product and a platelet product after the irradiation of the ultraviolet ray.

7. A blood product comprising red blood cells, platelets, a hemolysis inhibitor and a surfactant having an HLB value of 13 or greater and having the number of oxyethylene groups of a hydrophilic portion in the molecular structure of 20 or greater, wherein a pathogen in the blood product is inactivated.

8. A storage solution for a blood cell comprising a hemolysis inhibitor and a surfactant having an HLB value of 13 or greater and having the number of oxyethylene groups of a hydrophilic portion in the molecular structure of 20 or greater, wherein
the storage solution is used by being mixed with the blood sample in the method for producing set forth in any one of claims 1 to 6.
